(19) Europäisches Patentamt
European Patent Office
Office européen des brevets

(11) EP 1 013 642 B1

(12) **EUROPÄISCHE PATENTSCHRIFT**

(45) Veröffentlichungstag und Bekanntmachung des Hinweises auf die Patenterteilung:
**09.11.2005 Patentblatt 2005/45**

(51) Int Cl.[7]: **C07C 323/25**, C07F 13/00, C07H 21/00, A61K 51/10, A61K 51/08, A61K 51/04

(21) Anmeldenummer: **99250440.7**

(22) Anmeldetag: **16.12.1999**

(54) **Chelatoren sowie deren Tricarbonyl-Komplexe mit Technetium und Rhenium**

Chelating agents and the Technetium and Rhenium tricarbonyl complexes thereof

Agents chélatisants et leurs complexes avec Rhenium ou Technetium tricarbonyle

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **21.12.1998 DE 19860289**

(43) Veröffentlichungstag der Anmeldung:
**28.06.2000 Patentblatt 2000/26**

(73) Patentinhaber: **Schering Aktiengesellschaft**
**13353 Berlin (DE)**

(72) Erfinder:
- **Hilger, Christoph Stephan, Dr.**
  **13353 Berlin (DE)**
- **Berndorff, Dietmar, Dr.**
  **16540 Hohen-Neuendorf (DE)**
- **Blume, Friedhelm, Dr.**
  **13505 Berlin (DE)**
- **Dinkelborg, Ludger, Dr.**
  **13465 Berlin (DE)**
- **Heldmann, Dieter, Dr.**
  **13509 Berlin (DE)**

(56) Entgegenhaltungen:
**WO-A-95/05814** **WO-A-98/48848**

- **DATABASE CHEMABS [Online] CHEMICAL ABSTRACTS SERVICE, COLUMBUS, OHIO, US ABE, YOSHIRO ET AL: "Synthesis and antimicrobial properties of N-substituted amino acid-type amphoterics containing a thio ether linkage" retrieved from STN Database accession no. 85:110387 XP002131480 & YUKAGAKU (1976), 25(7), 419-23,**
- **DATABASE CHEMABS [Online] CHEMICAL ABSTRACTS SERVICE, COLUMBUS, OHIO, US OKADA, HISASHI ET AL: "Composition for processing silver halide color photographic material and photographic processing method" retrieved from STN Database accession no. 118:112883 XP002131481 & JP 04 204533 A (FUJI PHOTO FILM CO., LTD., JAPAN) 24. Juli 1992 (1992-07-24)**
- **DATABASE CHEMABS [Online] CHEMICAL ABSTRACTS SERVICE, COLUMBUS, OHIO, US OKEY, R. W. ET AL: "Predicting stability constants of various chelating agents using QSAR technology" retrieved from STN Database accession no. 129:85331 XP002131482 & EMERGING TECHNOL. HAZARD. WASTE MANAGE. 7, [PROC. I&EC DIV. ACS SYMP.] (1997), MEETING DATE 1996, 49-68. EDITOR(S): TEDDER, D. WILLIAM;POHLAND, FREDERICK G. PUBLISHER: PLENUM, NEW YORK, N. Y.,**
- **DATABASE CHEMABS [Online] CHEMICAL ABSTRACTS SERVICE, COLUMBUS, OHIO, US KISHIMOTO, SHINZO ET AL: "Silver halide color photographic materials" retrieved from STN Database accession no. 107:165332 XP002131483 & JP 61 252552 A (FUJI PHOTO FILM CO., LTD., JAPAN) 10. November 1986 (1986-11-10)**
- **YAMAMURA, NORIO ET AL: "Technetium-99m-Labeled Medium-Chain Fatty Acid Analogs Metabolized by.beta.-Oxidation: Radiopharmaceutical for Assessing Liver Function" BIOCONJUGATE CHEM., Bd. 10, Nr. 3, 1999, Seiten 489-495, XP002137894**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 013 642 B1

Printed by Jouve, 75001 PARIS (FR)

## Beschreibung

Die Erfindung liegt auf dem Gebiet der Radiopharmaka und beschreibt neue Chelatoren sowie deren Tricarbonyl-Komplexe mit Technetium und Rhenium.

Komplexe mit radioaktiven Metallen werden in der Radiodiagnostik und Radiotherapie schon sehr lange verwendet. Am häufigsten wird das Radionuklid Technetium-99m verwendet, da es aufgrund seiner günstigen physikalischen Eigenschaften (keine Korpuskularstrahlung, geringe Halbwertszeit von 6.02 h, gute Detektierbarkeit durch seine 140 KeV γ-Strahlung), seiner geringen biologischen Halbwertszeit sowie seiner breiten Verfügbarkeit besonders gut für eine in-vivo-Anwendung geeignet ist. Zur Synthese von Technetium-99m-Komplexen wird Pertechnetat zunächst aus einem Nuklidgenerator gewonnen und durch Verwendung geeigneter Reduktionsmittel (z.B. $SnCl_2$) in eine niedrigere Oxidationsstufe überführt, die anschließend durch einen geeigneten Chelator stabilisiert wird. Da Technetium in einer Reihe von Oxidationsstufen (+7 bis -1) vorliegen kann, welche die pharmakologischen Eigenschaften durch Veränderungen der Ladung eines Komplexes stark verändern können, ist es notwendig, Chelatoren bzw. Komplexliganden für Technetium-99m bereitzustellen, die Technetium sicher, fest und stabil in einer definierten Oxidationsstufe binden können. Die Chelatoren verhindern in vivo ablaufende Redoxprozesse bzw. Technetiumfreisetzungs-Reaktionen. Derart unerwünschte Reaktionen erschweren eine sichere Diagnostik von Erkrankungen, da die Anreicherung des Radiopharmakons in Läsionen, die Pharmakokinetik des Radiopharmakons und dessen Ausscheidung durch seine Metabolite bestimmt wird.

Als geeignete Komplexbildner für Technetium- und Rheniumisotope gelten z.B. zyklische Amine [Troutner, D. E. et al., J. Nucl. Med. 21, 443 (1980)], die aber den Nachteil haben, daß sie erst ab einem pH-Wert > 9 in der Lage sind, Technetium-99m in guten Ausbeuten zu binden. $N_20_2$-Systeme [Pillai, M. R. A., Troutner, D. E. et al., Inorg. Chem. 29, 1850 (1990)] befinden sich in der klinischen Anwendung. Nichtzyklische $N_4$-Systeme, wie z.B. das HMPAO, haben nur eine geringe Komplexstabilität. Tc-99m-HMPAO muß wegen seiner Instabilität [Ballinger, J. R. et al., Appl. Radiat. Isot 42, 315 (1991); Billinghurst, M. W. et al., Appl. Radiat. Isot. 42, 607 (1991)] sofort nach seiner Markierung appliziert werden, damit der Anteil an Zerfallsprodukten, die eine andere Pharmakokinetik und Ausscheidung als das Diagnostikum besitzen, klein gehalten werden kann. Die radiochemischen Verunreinigungen erschweren die Erkennung der zu diagnostizierenden Erkrankungen. $N_2S_2$-Chelatoren [Bormans, G. et al., Nucl. Med. Biol. 17, 499 (1990)] wie z.B. Ethylendicystein [Verbruggen, A. M. et al., J. Nucl. Med. 33, 551 (1992)] erfüllen zwar die Forderung nach hinreichender Stabilität des entsprechenden Technetium-99m-Komplexes, bilden aber erst ab einem pH-Wert >9 Radiodiagnostika mit einer Reinheit über 69%. $N_3S$-Systeme (Fritzberg, A., EPA 0 173 424 und EPA 0 250 013) bilden zwar stabile Technetium-99m-Komplexe, müssen aber zum Einbau des Radioisotops auf Temperaturen von ca. 100°C erhitzt werden. Ein weiterer Nachteil der $N_2S_2$ und $N_3S$-Systeme besteht darin, daß diese teilweise rasch und ohne spezifische Anreicherung vom Organismus ausgeschieden werden, so daß diese nur als Nierenfunktionsdiagnostika in der Klinik Anwendung finden. Sie besitzen daher nur eine eingeschränkte Verwendbarkeit. Die Kopplung solcher Chelate bzw. Chelatbildner an sich selektiv in Krankheitsherden anreichernden Substanzen ist mit einfachen Mitteln nicht zu lösen, so daß sich diese im allgemeinen unspezifisch im Organismus verteilen.

In den letzten Jahren ist das Verlangen nach sich spezifisch in erkrankten Geweben anreichernden Radiodiagnostika und Radiotherapeutika gestiegen. Dies kann erreicht werden, wenn Komplexbildner leicht an sich selektiv in Läsionen anreichernde Substanzen gekoppelt werden können und dabei ihre günstigen Komplexierungseigenschaften nicht verlieren. Da aber häufig nach Kopplung eines Komplexbildners an ein solches Molekül eine Abschwächung der Komplexstabilität beobachtet wird, erscheinen die bisherigen Ansätze zur Kopplung von Chelatbildnern an sich selektiv anreichernde Substanzen wenig zufriedenstellend. Die Ursache liegt darin begründet, daß ein diagnostisch nicht tolerierbarer Anteil des Isotops aus dem Konjugat in vivo freigesetzt wird [Brechbiel, M. W. et al., Inorg. Chem. 25, 2772 (1986)]. Es ist deshalb notwendig, bifunktionelle Komplexbildner bereitzustellen, die sowohl funktionelle Gruppen zur stabilen Bindung des gewünschten Metallions als auch eine oder mehrere weitere funktionelle Gruppen zur Bindung des selektiv anreichernden Moleküls tragen. Solche bifunktionellen Liganden ermöglichen eine spezifische, chemisch definierte Bindung von Technetium- oder Rhenium-Isotopen an verschiedenste biologische Materialien; auch dann, wenn ein sogenanntes Prelabeling durchgeführt wird. Es wurden einige Chelatbildner beschrieben, die z.B. an monoklonale Antikörper (z.B. EP 247 866 und EP 188 256) oder Fettsäuren (EP 200 492) gekoppelt wurden. Als Chelatbildner wurden jedoch die bereits erwähnten $N_2S_2$-Systeme verwendet, die aufgrund ihrer geringen Stabilität wenig geeignet sind. Da sowohl die Eigenschaften der sich selektiv anreichernden Substanzen sowie die Mechanismen, nach denen sie in Läsionen angereichert werden, sehr unterschiedlich sind, ist es weiterhin notwendig, den kopplungsfähigen Chelatbildner zu variieren und den physiologischen Anforderungen des Kopplungspartners hinsichtlich seiner Lipo- und Hydrophilie, Membranpermeabilität bzw. -impermeabilität anpassen zu können.

Um die referierten Nachteile und Limitierungen der etablierten Chelatoren sowie deren Konjugate mit Biomolekülen, die selektiv in erkrankten Geweben akkumulieren, zu umgehen, ist in den letzten Jahren versucht worden, Tricarbonyl-Technetium-I- und Tricarbonyl-Rhenium-I-Verbindungen für das Labeling von solchen Biomolekülen zu verwenden (Lit. R. Alberto et al., Achievements and Prospects of New Radiotracers, 1997, C3, S. 57 (Abstracts)). Da

jedoch Tricarbonyl-Technetium-I-triaqua-Ionen und Tricarbonyl-Rhenium-I-triaqua-Ionen mit hoher Stabilität, unspezifisch und schnell von Serumproteinen gebunden werden, konnten bisher keine hinreichend stabile Konjugate zwischen sich selektiv in erkrankten Geweben anreichernden Substanzen mit Tricarbonyl-Technetium-I- und Tricarbonyl-Rhenium-I-Komplexen synthetisiert werden.

Die Internationale Patentanmeldung WO 98/48848 beschreibt eine allgemeine Methode zur Herstellung von Tricarbonyl-Technetium-I- und Tricarbonyl-Rhenium-I-Komplexen. Spezielle Chelatoren, die an Biomoleküle gekoppelt werden können und mit deren Hilfe besonders stabile Komplexe erhalten werden, werden in dieser Anmeldung nicht genannt.

Aufgabe der Erfindung war es daher, stabile Tricarbonyl-Technetium-I- und Tricarbonyl-Rhenium-I-Komplexe zu entwickeln, die an unterschiedliche, sich selektiv in erkrankten Geweben anreichernden Verbindungen gekoppelt werden können. Weitere Aufgabe der Erfindung war es, solche koppelbaren Chelatoren oder Komplexe bereitzustellen, die über eine große chemische Variationsbreite der Substituenten verfügen, um diese den oben referierten Erfordernissen anpassen zu können. Ein weiterer Aspekt der Erfindung betrifft Verfahren zur Herstellung der Verbindungen sowie die die Verbindungen enthaltenden pharmazeutische Mittel.

**Diese Aufgabe wird durch die Verbindungen der allgemeinen Formel (I) gelöst**

$$Y\text{-}CR^1R^2\text{-}(CR^3R^4)_n\text{-}N(CR^5R^6\text{-}COOR^7)\text{-}CR^8R^9\text{-}CO\text{-}X \qquad (I)$$

worin
n für die Zahlen 0,1 oder 2 steht;
$R^1$, $R^2$, $R^3$, $R^4$, $R^5$, $R^6$, $R^8$ und $R^9$ gleich Wasserstoffatome sind;
$R^7$ für ein Wasserstoffatom oder ein Alkali-Kation oder ein Erdalkali-Kation oder ein primäres, sekundäres oder tertiäres Ammoniumion steht;
X einen Rest $NR^{10}R^{11}$ bedeutet, worin $R^{10}$ und $R^{11}$ gleich oder unterschiedlich sind und jeweils ein Wasserstoffatom oder einen unverzweigten, verzweigten, cyclischen oder polycyclischen Alkyl- mit bis zu 60 C-Atomen, Alkenyl- mit bis zu 60 C-Atomen, Polyalkenyl- mit bis zu 60 C-Atomen, Alkinyl mit bis zu 60 C-Atomen, $C_5$-$C_{60}$-Polyalkinyl-, $C_5$-$C_{60}$-Aryl-, Alkylaryl- mit bis zu 60 C-Atomen oder Arylalkylrest mit bis zu 60 C-Atomen darstellen, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{10}$ und/oder $R^{11}$ gleich oder unterschiedlich sind und jeweils ein Wasserstoffatom, einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellen;
Y für einen $R^{12}$-S-Rest steht, worin $R^{12}$ ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinylmit bis zu 60 C-Atomen, $C_5$-$C_{60}$-Polyalkinyl-, $C_5$-$C_{60}$-Aryl-, Alkylaryl- mit bis zu 60 C-Atomen oder Arylalkylrest mit bis zu 60 C-Atomen darstellt, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{12}$ einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellt;
sowie deren Komplexe mit Tricarbonyl-Technetium-I- oder Tricarbonyl-Rhenium-I-Resten der entsprechenden Radioisotope.

Weiter sind solche Verbindungen bevorzugt, bei denen $R^7$ für ein Wasserstoffatom, ein Alkali-Kation oder ein Erdalkali-Kation steht.

Der Rest Y steht bevorzugt für einen Rest $R^{12}$-S, worin $R^{12}$ bevorzugt für einen unverzweigten oder verzweigten $C_1$-$C_{60}$-Alkylrest steht. Besonders bevorzugt sind solche Verbindungen, bei denen der Rest $R^{12}$ eine $C_1$-$C_{10}$-Alkylkette darstellt.

Der Rest X steht bevorzugt für eine Gruppe $NR^{10}R^{11}$, worin $R^{10}$ und $R^{11}$ gleich oder unterschiedlich sind und jeweils ein Wasserstoffatom, einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellen, oder für eine Gruppe $NR^{10}R^{11}$, worin $R^{10}$ und $R^{11}$ gleich oder unterschiedlich sind und jeweils für ein Wasserstoffatom, einen unverzweigten oder verzweigten $C_1$-$C_{60}$-Alkylrest stehen.

Die Herstellung der erfindungsgemäßen Verbindungen der allgemeinen Formel (I) erfolgt dadurch, daß man Verbindungen der allgemeinen Formel (II) mit Verbindungen der allgemeinen Formel (III) gemäß nachfolgendem Reaktionsschema

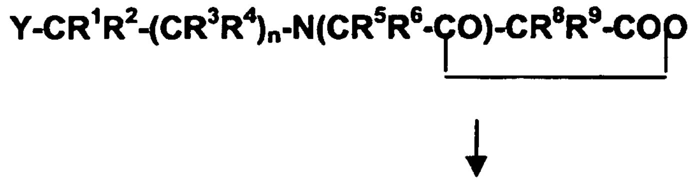

(II) + HX (III)

$$Y\text{-}CR^1R^2\text{-}(CR^3R^4)_n\text{-}N(CR^5R^6\text{-}COOR^7)\text{-}CR^8R^9\text{-}CO\text{-}X \quad (I)$$

umsetzt,
worin
n für die Zahlen 0,1, oder 2 steht;
$R^1$, $R^2$, $R^3$, $R^4$, $R^5$, $R^6$, $R^7$, $R^8$ und $R^9$ die vorstehend angegebene Bedeutung haben und H in HX für ein Proton steht;
X einen Rest $NR^{10}R^{11}$ bedeutet, worin $R^{10}$ und $R^{11}$ gleich oder unterschiedlich sind und jeweils ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinyl- mit bis zu 60 C-Atomen, $C_5$-$C_{60}$-Polyalkinyl-, $C_5$-$C_{60}$-Aryl-, Alkylaryl- mit bis zu 60 C-Atomen oder Arylalkylrest mit bis zu 60 C-Atomen darstellen, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{10}$ und/oder $R^{11}$ gleich oder unterschiedlich sind und jeweils ein Wasserstoffatom, einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellen,
Y für einen $R^{12}$-S-Rest steht, worin $R^{12}$ ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinylmit bis zu 60 C-Atomen, $C_5$-$C_{60}$-Polyalkinyl-, $C_5$-$C_{60}$-Aryl-, Alkylaryl- mit bis zu 60 C-Atomen oder Arylalkylrest mit bis zu 60 C-Atomen darstellt, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{12}$ einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellt.

Die Herstellung der erfindungsgemäßen Technetium-99m- oder Re-Tricarbonylkomplexe erfolgt durch Reaktion der vorsynthetisierten Technetiumtricarbonyl- bzw. Rheniumtricarbonyl-Precursor [R. Alberto et al., Achievements and Prospects of New Radiotracers, 1997, C3, S. 57 (Abstracts)] mit den erfindungsgemäßen Tridentaten.

Ein weiterer Gegenstand der Erfindung sind radiopharmazeutische Zusammensetzungen zur nicht invasiven in-vivo-Darstellung von Rezeptoren und rezeptorhaltigem Gewebe, die eine Verbindung der allgemeinen Formel (I) sowie gegebenenfalls in der Galenik übliche Zusätze enthalten. Die radiopharmazeutische Zusammensetzung wird in einer Menge von 0,1mCi bis 1Ci, bevorzugt von 1mCi bis 500mCi pro 70 kg Körpergewicht einem Patienten verabreicht. Die vom Patienten abgegebene Strahlung wird mittels einer Gamma-Kamera aufgezeichnet.

Überraschenderweise zeigten die synthetisierten und mit Tc-99m-Tricarbonyl- oder Re-Tricarbonyl-markierten Chelate eine höhere Stabilität als vergleichbare $N_3S$- und $N_2S_2$-Systeme, die in der Literatur beschrieben wurden. So wurden z.B. bei einer erfindungsgemäßen Substanz (Beispiel 2b) keine Zersetzungsprodukte nach 26 stündiger Inkubation im Serum beobachtet. Die Komplexe sind so stabil, daß bei Raumtemperatur kein Austausch der Chelatoren gegen Histidin erfolgt. Die in der vorliegenden Erfindung beschriebenen Chelate und Tricarbonyl-Tc-99m- und Re-Tricarbonyl-Komplexe sind damit eindeutig besser für diagnostische und therapeutische Zwecke geeignet als die bisher bekannten Systeme. Ein besonderer Vorteil der erfindungsgemäßen Verbindungen besteht darin, daß deren Synthesen ohne Verwendung von Schwefelschutzgruppen geführt werden können. Dies macht deren Synthese sehr einfach, und zusätzlich bieten speziell solche erfindungsgemäß beschriebenen Verbindungen den Vorteil, daß nach radiochemischer Markierung keine weiteren Fremdmoleküle in den zur Radiodiagnostik bzw. Radiotherapie intravenös zu applizierenden Lösungen enthalten sind. Solche Fremdmoleküle stören häufig die Biodistribution des Radiopharmakons und können den diagnostischen Inforrnationsgehalt der SPECT-Bildgebung nachteilig beeinflussen. Außerdem können die Markierungen an solchen Liganden bzw. deren Kopplungsprodukte an sich selektiv in erkrankten Geweben anreichernden Substanzen unter sehr milden Bedingungen vorgenommen werden. So gelingt die Markierung der erfindungsgemäßen Liganden bzw. der Kopplungsprodukte an sich selektiv in erkrankten Geweben anreichernden Substanzen bei Raumtemperatur und bei physiologischem pH-Wert, ohne daß vorher unter Einwirkung von Basen, Säuren oder anderen dem Fachmann bekannten Hilfsstoffen die Schutzgruppen abzuspalten wären. Dies gewährleistet, daß durch solche Hilfsstoffe die häufig sehr empfindlichen, sich selektiv in erkrankten Geweben anreichernden Substanzen nicht chemisch verändert werden, was häufig deren selektive Anreicherung in erkranktem Gewebe herabsetzt und somit den Informationsgehalt der SPECT-Aufnahmen nachteilig beeinflussen würde.

Die Kopplung an sich selektiv in erkrankten Geweben anreichernden Substanzen erfolgt nach den dem Fachmann bekannten Methoden [z.B. Fritzberg et al.; J. Nucl. Med. 26, 7 (1987)], beispielsweise durch Reaktion von elektrophilen Gruppen des erfindungsgemäßen Komplexliganden mit nukleophilen Zentren der sich selektiv in erkrankten Geweben anreichernden Substanzen. Ansonsten werden nukleophile Gruppen des Chelators mit elektrophilen Gruppen der sich selektiv in erkrankten Geweben anreichernden Substanzen gekoppelt.

Als Kopplungspartner sind u.a. verschiedene Biomoleküle vorgesehen sowie biologische Liganden, die an spezifische Rezeptoren binden und so ein in ihrer Rezeptordichte verändertes Gewebe erkennen können. Hierzu gehören z.B. modifizierte oder unmodifizierte Peptide, unmodifizierte oder modifizierte Proteine, Steroidhormone oder deren Derivate, Wachstumsfaktoren, Neurotransmitter, modifizierte DNA- oder RNA-Oligonukleotide sowie modifizierte oder unmodifizierte Aptamere oder PNA-Moleküle.

Die Herstellung der erfindungsgemäßen pharmazeutischen Mittel erfolgt in an sich bekannter Weise, indem man die erfindungsgemäßen Komplexbildner gegebenenfalls unter Zugabe der in der Galenik üblichen Zusätze in wäßrigem Medium löst und anschließend sterilfiltriert. Geeignete Zusätze sind beispielsweise physiologisch unbedenkliche Puffer, Zusätze von Elektrolyten (z.B. Natriumchlorid) und gegebenenfalls Stabilisatoren. Das erfindungsgemäße pharmazeutische Mittel liegt in Form einer Lösung oder in lyophilisierter Form vor und wird kurz vor der Applikation mit einer Lösung des Tc-99m-Tricarbonyl- oder Re-Tricarbonyl-Precursor versetzt.

Bei der nuklearmedizinischen in-vivo-Anwendung werden die erfindungsgemäßen Mittel intravenös, intraarteriell, peritoneal oder intratumoral injiziert.

Die nachfolgenden Beispiele dienen der näheren Erläuterung des Erfindungsgegenstandes.

**BEISPIEL 1: HERSTELLUNG EINES CHELATORS; Vergleichsbeispiel**

**Beispiel 1a**

**N,N-Bis-(tert.-Butoxycarboxy-methyl)-S-ethyl-2-mercaptoethylamin**

3.52g (10mmol) N,N-Bis-(tert.-Butoxycarboxy-methyl)-2-brom-ethylamin werden 50ml absolutem Dichlormethan gelöst und mit 1.29g (10mmol) Diisopropylethylamin und 0.621g (10mmol) Ethylmercaptan versetzt. Das Reaktiongemisch wird über Nacht unter Rückfluß erhitzt. Anschließend wird die Lösung zweimal mit halbgesättigter, wäßriger Natriumhydrogencarbonatlösung extrahiert. Die organische Phase wird über Natriumsulfat getrocknet und das Lösungsmittel im Vakuum verdampft. Der Rückstand wird an Kieselgel chromatographiert (Eluent: Dichlormethan/Methanol 99:1).

Ausbeute: 1.82g (54.6%), weißer Schaum.

| Elementaranalyse: | | | | |
|---|---|---|---|---|
| C 57.63 | H 9.37 | N 4.20 | O 19.19 | S 9.61 |
| C 57.43 | H 9.57 | N 4.12 | O | S 9.32 |

**Beispiel 1b**

**N,N-Bis-(Hydroxycarboxy-methyl)-S-ethyl-2-mercaptoethylaminhydrochlorid**

1.67g (5mmol) N,N-Bis-(tert.-Butoxycarboxy-methyl)-S-ethyl-2-mercaptoethylamin werden in 12ml Tetrahydrofuran gelöst und mit 6ml konzentrierter Salzsäure versetzt. Man rührt das resultierende Reaktionsgemisch 1h bei Raumtemperatur und engt im Vakuum ein. Der Rückstand wird mit 10ml absolutem Diethylether verrieben. Anschließend wird das Produkt abfiltriert und im Feinvakuum getrocknet.
Ausbeute: 1.52g (59%), weißes Pulver.

| Elementaranalyse: | | | | | |
|---|---|---|---|---|---|
| C 37.28 | H 6.26 | N 5.44 | O 24.83 | S 12.44 | Cl 13.76 |
| C 37.02 | H 6.43 | N 5.16 | O | S 12.20 | Cl 13.52 |

**Beispiel 1c**

**N-(S-Ethyl-2-mercapto-eth-1-yl)-1-aza4-oxa-3,5-dioxo-cyclohexanhydrochlorid**

773.2mg (3mmol) N,N-Bis-(Hydroxycarboxy-methyl)-S-ethyl-2-mercaptoethylamin-hydrochlorid werden in 25ml absolutem Dimethylformamid gelöst. Man tropft 619mg (2mmol) Dicyclohexylcarbodiimid, gelöst in 5ml absolutem Dimethylformamid, zum Reaktionsansatz. Die resultierende Lösung wird 1h bei Raumtemperatur gerührt. Nach Abfiltrieren des ausgefallenen Dicyclohexylharnstoffes kann die Lösung des zyklischen Anhydrids zur Kopplung an Aminogruppen-tragende Moleküle/Biomoleküle verwendet werden. Die Lösung ist 0.1 molar an zyklischem Anhydrid.

**BEISPIEL 2: HERSTELLUNG EINES TRICARBONYL-TECHNETIUM-KOMPLEXES UND EINES TRICARBONYL-RHENIUM-KOMPLEXES MIT EINEM AN PROPYLAMIN GEKOPPELTEN CHELATOREN DER ALLGEMEINEN FORMEL (I)**

**Beispiel 2a:**

**N-(Carboxymethyl)-N-(N'-propyl-carbamido-methyl)-S-ethyl-2-mercaptoethylamin**

30ml der unter Beispiel 1c beschriebenen Lösung des N-(S-Ethyl-2-mercaptoeth-1-yl)-1-aza-4-oxa-3,5-dioxo-cyclohexan-hydrochlorids werden mit 303.6mg (3mmol) Triethylamin und 177.3g (3mmol) Propylamin versetzt. Man rührt 1h bei Raumtemperatur, verdampft das Lösungsmittel im Vakuum und chromatographiert den Rückstand an Kieselgel (Eluent: Dichlormethan/Methanol 92:8).
Ausbeute: 617mg (78.4%), weißes Pulver.

| Elementaranalyse: | | | | |
|---|---|---|---|---|
| C 50.36 | H 8.45 | N 10.68 | O 18.29 | S 12.22 |
| C 50.18 | H 8.66 | N 10.41 | O | S 12.03 |

**Beispiel 2b:**

**Tc-99m-Tricarbonylkomplex von N-(Carboxymethyl)-N-(N'-propylcarbamido-methyl)-S-ethyl-2-mercaptoethylamin**

1mg N-(Carboxymethyl)-N-(N'-propyl-carbamido-methyl)-S-ethyl-2-mercaptoethylamin werden in 1ml 0.1 M Dinatriumphosphatpuffer (pH = 8.5) gelöst. Anschließend setzt man 370MBq $[^{99m}Tc(OH_2)_3(CO)_3]^+$-Lösung [hergestellt in Anlehnung an R. Alberto et al., Achievements and Prospects of New Radiotracers, 1997, C3, S. 57 (Abstracts)] hinzu und inkubiert 30min bei Raumtemperatur. Die radiochemische Ausbeute und die radiochemische Reinheit des Radiopharmakons werden mittels HPLC geprüft (>91 %).

**Beispiel 2c:**

**Stabilitätsuntersuchung des Tc-99m-Tricarbonylkomplexes von N-(Carboxymethyl)-N-(N'-propylcarbamido-methyl)-S-ethyl-2-mercaptoethylamin:**

Die nach Beispiel 2b hergestellte Lösung wird mit 10 mg Histidin versetzt und 1 h bei 50°C gerührt. Anschließend wird die radiochemische Zusammensetzung mit der HPLC auf einer RP18-Säule untersucht: Das Chromatogramm ist unverändert. Der zu Vergleichszwecken hergestellte Tricarbonylkomplex mit Histidin hat erheblich kürzere Retentionszeiten.

**Beispiel 2d:**

**Herstellung des Rhenium-Tricarbonylkomplexes von N-(Carboxymethyl)-N-(N'-propylcarbamidomethyl)-S-ethyl-2-mercaptoethylamin:**

49,3 mg Tetrabutylammoniumperrhenat und 139 mg Tetrabutylammoniumchlorid werden in 5 ml Diethylenglykol-dimethylether gelöst, die Lösung mit Kohlenmonoxid gesättigt und unter CO-Athmosphäre 1,5 ml einer 1-molaren Boran-Lösung in THF zugetropft. 4 Stunden wird bei einer Ölbadtemperatur von 110°C nachgerührt, dabei wird ein langsamer CO-Gasstrom durchgeleitet. Nach dem Abkühlen wird zu der so entstandenen Lösung eine Lösung von 39,3 mg N-(Carboxymethyl)-N-(N'-propylcarbamidomethyl)-S-ethyl-2-mercaptoethylamin in 1 ml THF zugetropft und anschließend 30 Minuten zum Rückfluß erwärmt. Die Reaktionsmischung wird eingeengt und der Rückstand durch Chromatografie mit dem Laufmittel 90% Methylenchlorid und 10% Methanol aufgereinigt.
Ausbeute 33,7 mg = 63,6%. Strukturnachweis spektroskopisch mit NMR, IR und MS.

**BEISPIEL 3: HERSTELLUNG EINES TRICARBONYL-TECHNETIUM-KOMPLEXES MIT EINEM AN EIN OLIGONUCLEOTID GEKOPPELTEN CHELATOREN; Vergleichsbeispiel**

**Beispiel 3a**

**5'-(6-Aminohexylphosphato)-*GGAGfUfCfUfUAGGfCAGfCGfCGfUfUfUfUfCGAGfCfUAfCfUfCfC*-3'-3'-dT (f: 2'-F)**

Das 33mer-Oligonukleotid *GGAGfUfCfUfUAGGfCAGfCGfCGfUfUfUfUfCGAGfCfUAfCfUfCfC (f: 2'-F),* wurde in 3'-3'-dT gecappter Form in üblicher Weise mittels eines Syntheseautomaten der Fa. Pharmacia hergestellt (siehe Oligonucleotides and Analogues, A Practical Approach, Ed. F. Eckstein, Oxford University Press Oxford, New York, Tokyo, 1991), wobei sich das Oligonukleotid noch auf der Säule des festen Trägers befand. Durch Reaktion mit Trichloressigsäure in Dichlormethan wurde die 5'-Hydroxylgruppe freigesetzt. Die Beladung der Säule betrug ca. 10mg an 34-mer. Zum Anknüpfen des Aminolinkers wurde die Säule mit einer Lösung von 50µmol β-Cyanoethyl-N,N-diisopropylamino-6-(trifluoracetamido)-1-hexyl-phosphoramidit [hergestellt nach Nucl. Acids. Res. 16, 2659-2669 (1988)] in Gegenwart von Tetrazol umgesetzt. Die Oxidation des gebildeten Phosphits zum voll geschützten Phosphotriester erfolgte mit Iod in Tetrahydrofuran/Pyridin/Wasser. Die Abspaltung des Oligonukleotids vom Träger und die Schutzgruppenabspaltung geschah nach Standardverfahren (siehe Oligonucleotides and Analogues, A Practical Approach, Ed. F. Eckstein, Oxford University Press Oxford, New York, Tokyo, 1991, S. 36). Das rohe Oligonukleotid wurde durch Dialyse von TBAF-Spuren befreit und anschließend durch RP-Chromatographie aufgereinigt. Die Titelverbindung wurde durch Lyophilisieren gewonnen.
Ausbeute: 6.5mg, weißes Pulver.

**Beispiel 3b**

**Konjugation des Oligonukleotids 3a mit 1c: 5'-[Et-S-$CH_2$-$CH_2$-N($CH_2$-COOH)-$CH_2$-CO-NH-$(CH_2)_6$-O-phosphato) -*GGAGfUfCfUfUAGGfCAGfCGfCGfUfUfUfUfCGAGfCfUAfCfUfCfC*-3'-3'-dT (f: 2'-F)**

5mg des in Beispiel 1d erhaltenen Oligonukleotids werden in 1ml $NaHCO_3$/$Na_2CO_3$-Puffer (pH 9, 0.2M) gelöst und mit 100µl der unter Beispiel 1c hergestellten Lösung des N-(S-Ethyl-2-mercapto-eth-1-yl)-1-aza-4-oxa-3,5-dioxocyclohexan-hydrochlorid in DMF versetzt. Man rührt 3h bei Raumtemperatur, stellt den pH-Wert durch Zugabe von 0.01 M Salzsäure auf 7.2 ein und fällt das Konjugat durch Zugabe von 9ml Ethanol bei -70°C aus. Die Reinigung der Titelverbindung geschah durch RP-Chromatographie.
Ausbeute: 1.1mg, weißes Pulver.

**Beispiel 3c**

**Tc-99m-Tricarbonylkomplex des Oligonukleotids 3b**

100µg 5'-[Et-S-$CH_2$-$CH_2$-N($CH_2$-COOH)-$CH_2$-CO-NH-$(CH_2)_6$-O-phosphato)-*GGAGfUfCfUfUAGGfCAGfCGfCGfUfUfUfUfCGAGfCfUAfCfUfCfC*-3'-3'-dT (f: 2'-F) werden in 150µl Dinatriumhydrogenphosphat-Puffer (pH = 8.5, 0.1 M) gelöst. Man versetzt mit 184MBq $[^{99m}Tc(OH_2)_3(CO)_3]^+$-Lösung [hergestellt in Anlehnung an R. Alberto et al., Achievements and Prospects of New Radiotracers, 1997, C3, S. 57 (Abstracts)] und läßt das Reaktionsgemisch für 30min bei Raumtemperatur stehen. Die Markierungsausbeute wird mittels HPLC- und PAGE-Analyse bestimmt und ist >90%.

**BEISPIEL 4 : HERSTELLUNG EINES TRICARBONYL-TECHNETIUM-KOMPLEXES MIT EINEM AN BOVINSERUMALBUMIN GEKOPPELTEN CHELATOREN; Vergleichsbeispiel**

**Beispiel 4a**

**Konjugation des Proteins Bovinserumalbumin (BSA) mit 1c: Et-S-$CH_2$-$CH_2$-N($CH_2$-COOH)-$CH_2$-CO-BSA**

5mg BSA werden in 500µl 0.1M Dinatriumphosphatpuffer (pH = 8.5) gelöst. Man setzt 100µl der unter Beispiel 1c hergestellten Lösung des N-(S-Ethyl-2-mercapto-eth-1-yl)-1-aza-4-oxa-3,5-dioxo-cyclohexan-hydrochlorid in DMF hinzu und rührt 2.5h bei Raumtemperatur. Das Konjugat wird durch Dialyse gegen PBS von nicht umgesetzten 1c befreit . Die so präparierte Lösung von 4a kann ohne weitere Reinigung weiterverwendet werden.
Ausbeute: 3.5mg Konjugat pro 1.3ml PBS

**Beispiel 4b**

**Tc-99m-Tricarbonylkomplex des Proteins 4a**

1mg (371µl) des Konjugates 4a werden mit 180MBq $[^{99m}Tc(OH_2)_3(CO)_3]^+$-Lösung [hergestellt in Anlehnung an R. Alberto et al., Achievements and Prospects of New Radiotracers, 1997, C3, S. 57 (Abstracts)] versetzt. Man läßt das Reaktionsgemisch für 60min bei Raumtemperatur stehen. Die Markierungsausbeute wird mittels SE-HPLC- bestimmt und ist >90%.

**BEISPIEL 5 : HERSTELLUNG EINES TRICARBONYL-TECHNETIUM-KOMPLEXES MIT EINEM AN EIN PEPTID GEKOPPELTEN CHELATOREN; Vergleichsbeispiel**

**Beispiel 5a**

**Konjugation des Peptids Phe-(D-Trp)-Leu-Asp-Ile-Ile-Trp-OH mit 1c: Et-S-$CH_2$-$CH_2$-N($CH_2$-COOH) -$CH_2$-CO-Phe-(D-Trp)-Leu-Asp-Ile-Ile-Trp-OH**

5mg des Peptids Phe-(D-Trp)-Leu-Asp-Ile-Ile-Trp-OH (hergestellt in Analogie zu E. Atherton & R.C. Sheppard, Solid Phase Peptide Synthesis, IRL PRESS 1989, Oxford) werden in 0.5ml absolutem Dimethylformamid gelöst und mit 1.52mg Triethylamin versetzt. Anschließend gibt man 100µl der unter Beispiel 1c hergestellten Lösung des N-(S-Ethyl-2-mercapto-eth-1-yl)-1-aza-4-oxa-3,5-dioxo-cyclohexan-hydrochlorid in DMF hinzu und rührt 3.5h bei Raumtemperatur. Das Konjugat wird durch RP-Chromatographie aufgereinigt und durch Lyophilisieren isoliert.
Ausbeute: 2.3mg (38.5%), weißes Pulver.
FAB-MS: 1197

**Beispiel 5b**

**Tc-99m-Tricarbonylkomplex des Peptids 5a**

1mg des Konjugates 5a werden mit 180MBq $[^{99m}Tc(OH_2)_3(CO)_3]^+$-Lösung [hergestellt in Anlehnung an R. Alberto et al., Achievements and Prospects of New Radiotracers, 1997, C3, S. 57 (Abstracts)] versetzt. Man läßt das Reaktionsgemisch für 30min bei Raumtemperatur stehen. Die Markierungsausbeute wird mittels SE-HPLC- bestimmt und ist >90%.

**BEISPIEL 6: HERSTELLUNG EINES CHELATORS; Vergleichsbeispiel**

**Beispiel 6a:**

**Herstellung von Di-t.-butyl-2,2'-{[2-(1H-pyrazol-1-yl)-ethyl]-imino}-diacetat**

1 g N-(2-Bromethyl)-iminodiessigsäure-di-t.butylester gelöst in 10 ml Methylenchlorid werden mit 213 mg Pyrazol versetzt und mit einer Mischung von 1ml 40%iger wässriger Tetrabutylammoniumhydroxidlösung und 1,14ml 5 molarer Natronlauge versetzt. Die Mischung wird 18 h bei Raumtemperatur unter Schutzgas heftig gerührt. Zur Aufarbeitung werden die Phasen getrennt, die organische Phase mit Wasser mehrmals gewaschen, mit Natriumsulfat getrocknet und eingeengt. Der Rückstand wird chromatographisch im Laufmittelsystem Methylenchlorid/Methanol gereinigt.
Ausbeute: 318 mg = 33% Öl
Nachweis spektroskopisch mit NMR, IR und MS

**Beispiel 6b:**

**Herstellung von [2-(1H-Pyrazol-1-yl)-ethylimino]-diessigsäure, Trifluoracetat**

330mg Di-t.-butyl-2,2'-{[2-(1 H-pyrazol-1-yl)-ethyl]-imino}-diacetat (Beispiel 6a) werden in 3,3 ml Trifluoressigsäure gelöst und 2 Stunden bei Raumtemperatur gerührt. Nach Einengen der Lösung am Hochvakuum wird der Rückstand mit Ether verrührt. Nach dem erneuten Trocknen am Hochvakuum verbleiben 281 mg Feststoff (85%)
Nachweis spektroskopisch mit NMR, IR und MS

**BEISPIEL 7: HERSTELLUNG EINES TRICARBONYL-TECHNETIUM-KOMPLEXES MIT EINEM AN PROPYLAMIN GEKOPPELTEN CHELATOREN; Vergleichsbeispiel**

**Beispiel 7a:**

**Herstellung von {2-(1H-pyrazol-1-yl)-ethylimino}-diessigsäuremonopropylamid**

268mg [2-(1 H-Pyrazol-1-yl)-ethylimino]-diessigsäure, Trifluoracetat (Beispiel 6b) werden in 20 ml DMF gelöst und mit 110 µl Triethylamin versetzt. Dann werden unter Schutzgasathmosphäre 162mg Dicyclohexylcarbodiimid gelöst in 3ml DMF langsam zugetropft und anschließend 16 Stunden bei Raumtemperatur gerührt. Zu der trüben Lösung werden 65 µl Propylamin gelöst in 3 ml DMF zugetropft, der Ansatz 1 Stunde gerührt, dann 65 µl Propylamin pur zugegeben und der Ansatz 2 Stunden nachgerührt. Der ausgefallene Feststoff wird abfiltriert, die Lösung eingeengt und der Rückstand chromatografisch im System Methylenchlorid/Methanol aufgereinigt. Ausbeute 48mg =23%
Nachweis spektroskopisch mit NMR, IR und MS

**Beispiel 7b:**

**Tc-99m-Tricarbonylkomplex von {2-(1H-pyrazol-1-yl)-ethylimino}diessigsäure-monopropylamid:**

2 mg {2-(1H-pyrazol-1-yl)-ethylimino}-diessigsäure-monopropylamid (Beispiel 7a) werden in 1 ml 0,1M Dinatriumphosphatpuffer (pH = 8,5) gelöst und mit 37 MBq $[^{99m}Tc(OH)_3(CO)_3]^+$-Lösung, hergestellt in Anlehnung an R. Alberto et al., Achievements and Prospects of New Radiotracers, 1997, C3, S.57 (Abstracts), versetzt. 30 min wird bei Raumtemperatur inkubiert und anschließend mittels HPLC analysiert: radiochemische Reinheit > 90%.

**Beispiel 7c:**

**Stabilitätsuntersuchung des Tc-99m-Tricarbonylkomplexes von {2-(1H-pyrazol-1-yl)-ethylimino}-diessigsäure-monopropylamid:**

Die nach Beispiel 7b hergestellte Lösung wird mit 20 mg Histidin versetzt und 1 h bei 50°C gerührt. Anschließend wird die radiochemische Zusammensetzung mit der HPLC auf einer RP18-Säule untersucht: Das Chromatogramm ist unverändert. Der zu Vergleichszwecken hergestellte Tricarbonylkomplex mit Histidin hat erheblich kürzere Retentionszeiten.

## Patentansprüche

1. **Verbindungen der allgemeinen Formel (I)**

$$\mathbf{Y\text{-}CR^1R^2\text{-}(CR^3R^4)_n\text{-}N(CR^5R^6\text{-}COOR^7)\text{-}CR^8R^9\text{-}CO\text{-}X} \qquad \mathbf{(I)}$$

worin
n für die Zahlen 0,1 oder 2 steht;
$R^1$, $R^2$, $R^3$, $R^4$, $R^5$, $R^6$, $R^8$ und $R^9$ gleich sind und Wasserstoff sind;

$R^7$ für eine Wasserstoffatom oder ein Alkali-Kation oder ein Erdalkali-Kation oder ein primäres, sekundäres oder tertiäres Ammoniumion steht;
X einen Rest $NR^{10}R^{11}$ bedeutet, worin $R^{10}$ und $R^{11}$ gleich oder unterschiedlich sind und jeweils ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinyl- mit bis zu 60 C-Atomen, $C_5$-$C_{60}$-Polyalkinyl-, $C_5$-$C_{60}$-Aryl-, Alkylaryl- mit bis zu 60 C-Atomen oder Arylalkylrest mit bis zu 60 C-Atomen darstellen, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{10}$ und/oder $R^{11}$ gleich oder unterschiedlich sind und jeweils ein Wasserstoffatom, einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellen;
Y für einen $R^{12}$-S-Rest steht, worin $R^{12}$ ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinylmit bis zu 60 C-Atomen, $C_5$-$C_{60}$-Polyalkinyl-, $C_5$-$C_{60}$-Aryl-, Alkylaryl- mit bis zu 60 C-Atomen oder $C_5$-$C_{60}$-Arylalkylrest mit bis zu 60 C-Atomen darstellt, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{12}$ einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellt;
sowie deren Komplexe mit Tricarbonyl-Technetium-I- oder Tricarbonyl-Rhenium-I-Resten der entsprechenden Radioisotope.

2. Verbindungen gemäß Anspruch 1, wobei $R^7$ für ein Wasserstoffatom oder ein Alkali-Kation oder ein Erdalkali-Kation steht.

3. Verbindungen gemäß Anspruch 1, wobei der Rest Y für einen Rest $R^{12}$-S steht, worin $R^{12}$ für einen unverzweigten oder verzweigten $C_1$-$C_{60}$-Alkylrest steht.

4. Verbindungen gemäß Anspruch 3, wobei der Rest $R^{12}$ eine $C_1$-$C_{10}$-Alkylkette darstellt.

5. Verbindungen gemäß Anspruch 1, wobei der Rest X für eine Gruppe $NR^{10}R^{11}$ steht, worin $R^{10}$ und $R^{11}$ gleich oder unterschiedlich sind und jeweils ein Wasserstoffatom, einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellen.

6. Verbindungen gemäß Anspruch 1, wobei der Rest X für eine Gruppe $NR^{10}R^{11}$ steht, worin $R^{10}$ und $R^{11}$ gleich oder unterschiedlich sind und jeweils für ein Wasserstoffatom, einen unverzweigten oder verzweigten $C_1$-$C_{60}$-Alkylrest stehen.

7. Verfahren zur Herstellung von Verbindungen der allgemeinen Formel (I) gemäß Anspruch 1, **dadurch gekennzeichnet, daß** man Verbindungen der allgemeinen Formel (II) mit Verbindungen der allgemeinen Formel (III) gemäß nachfolgendem Reaktionsschema

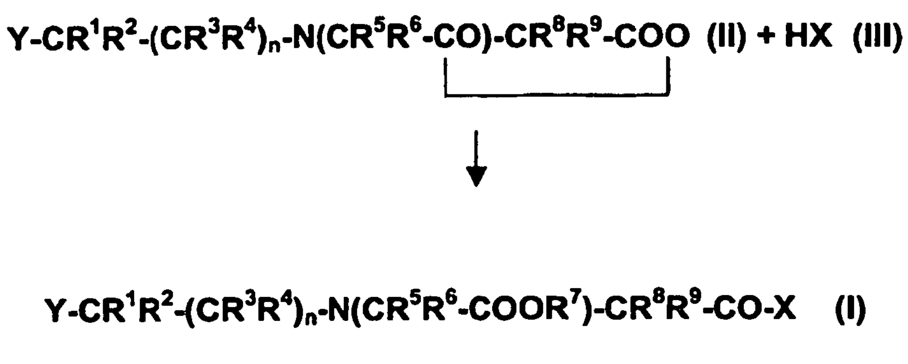

(II) + HX (III)

(I)

umsetzt,
worin
n für die Zahlen 0,1,oder 2 steht;
$R^1$, $R^2$, $R^3$, $R^4$, $R^5$, $R^6$, $R^8$ und $R^9$ gleich sind und Wasserstoff sind;
$R^7$ für eine Wasserstoffatom oder ein Alkali-Kation oder ein Erdalkali-Kation oder ein primäres, sekundäres oder tertiäres Ammoniumion steht;
und H in HX für ein Proton steht;
X einen Rest $NR^{10}R^{11}$ bedeutet, worin $R^{10}$ und $R^{11}$ gleich oder unterschiedlich sind und jeweils ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinyl- mit bis zu 60 C-Atomen, $C_5$-$C_{60}$-Polyalkinyl-, $C_5$-$C_{60}$-Aryl-, Alkylaryl- mit bis zu 60 C-Atomen oder Arylalkylrest mit bis zu 60 C-Atomen darstellen, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S P, As, Se unterbrochen und/oder substituiert ist; oder $R^{10}$ und/oder $R^{11}$ gleich oder unterschiedlich sind und jeweils ein Wasserstoffatom, einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellen;
Y für einen $R^{12}$-S-Rest steht, worin $R^{12}$ ein Wasserstoffatom oder einen unverzweigten, verzweigten, cyclischen oder polycyclischen $C_1$-$C_{60}$-Alkyl-, Alkenyl- mit bis zu 60 C-Atomen, Polyalkenyl- mit bis zu 60 C-Atomen, Alkinylmit bis zu 60 C-Atomen, $C_5$-$C_{6o}$-Polyalkinyl-, $C_5$-$C_{60}$-Aryl-, Alkylaryl- mit bis zu 60 C-Atomen oder Arylalkylrest mit bis zu 60 C-Atomen darstellt, welcher gegebenenfalls mit Hydroxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe 0, N, S, P, As, Se unterbrochen und/oder substituiert ist; oder $R^{12}$ einen Peptidrest, Proteinrest, einen modifizierten bzw. unmodifizierten DNA oder RNA-Oligonukleotidrest, einen modifizierten oder unmodifizierten Aptamer-Rest oder einen PNA-Rest darstellt.

8. Verwendung von Verbindungen der allgemeinen Formel (I) gemäß Anspruch 1 zur Herstellung von radiopharmazeutischen Zusammensetzungen zur nicht invasiven in-vivo-Darstellung von Rezeptoren und rezeptorhaltigem Gewebe.

9. Pharmazeutisches Mittel, **dadurch gekennzeichnet, daß** es eine Verbindung der allgemeinen Formel (I) gemäß Anspruch 1 in Lösung oder in lyophilisierter Form enthält.

## Claims

1. Compounds of the general formula (I)

$$\mathbf{Y\text{-}CR^1R^2\text{-}\ (CR^3R^4)_n\text{-}N(CR^5R^6\text{-}COOR^7)\text{-}CR^8R^9\text{-}CO\text{-}X} \qquad \mathbf{(I)}$$

in which
n is the number 0, 1 or 2;
$R^1$, $R^2$, $R^3$, $R^4$, $R^5$, $R^6$, $R^8$ and $R^9$ are equal to hydrogen atoms;
$R^7$ is a hydrogen atom or an alkali metal cation or an alkaline earth metal cation or a primary, secondary or tertiary ammonium ion;
X is a radical $NR^{10}R^{11}$ in which $R^{10}$ and $R^{11}$ are identical or different and are in each case a hydrogen atom or an unbranched, branched, cyclic or polycyclic $C_1$-$C_{60}$-alkyl radical, alkenyl radical having up to 60 C atoms, polyalkenyl radical having up to 60 C atoms, alkynyl radical having up to 60 C atoms, $C_5$-$C_{60}$-polyalkynyl radical, $C_5$-$C_{60}$-aryl radical, alkylaryl radical having up to 60 C atoms or arylalkyl radical having up to 60 C atoms, which is optionally substituted by hydroxy, oxo, carboxy, aminocarbonyl, alkoxycarbonyl, amino, aldehyde or alkoxy groups having up to 20 carbon atoms, and/or is optionally interrupted and/or substituted by one or more hetero atoms from the series O, N, S, P, As, Se; or $R^{10}$ and/or $R^{11}$ are identical or different and are in each case a hydrogen atom, a peptide residue, protein residue, a modified or unmodified DNA or RNA oligonucleotide residue, a modified or unmodified aptamer residue or a PNA residue;
Y is an $R^{12}$-S radical in which $R^{12}$ is a hydrogen atom or an unbranched, branched, cyclic or polycyclic $C_1$-$C_{60}$-alkyl radical, alkenyl radical having up to 60 C atoms, polyalkenyl radical having up to 60 C atoms, alkynyl radical having up to 60 C atoms, $C_5$-$C_{60}$-polyalkynyl radical, $C_5$-$C_{60}$-aryl radical, alkylaryl radical having up to 60 C atoms

or $C_5$-$C_{60}$-arylalkyl radical having up to 60 C atoms, which is optionally substituted by hydroxy, oxo, carboxy, aminocarbonyl, alkoxycarbonyl, amino, aldehyde or alkoxy groups having up to 20 carbon atoms, and/or is optionally interrupted and/or substituted by one or more hetero atoms from the series O, N, S, P, As, Se; or $R^{12}$ is a peptide residue, protein residue, a modified or unmodified DNA or RNA oligonucleotide residue, a modified or unmodified aptamer residue or a PNA residue;
and the complexes thereof with tricarbonyl-technetium-I or tricarbonyl-rhenium-I residues of the corresponding radioisotopes.

**2.** Compounds according to Claim 1, where $R^7$ is a hydrogen atom or an alkali metal cation or an alkaline earth metal cation.

**3.** Compounds according to Claim 1, where the radical Y is a radical $R^{12}$-S in which $R^{12}$ is an unbranched or branched $C_1$-$C_{60}$-alkyl radical.

**4.** Compounds according to Claim 3, where the radical $R^{12}$ is a $C_1$-$C_{10}$-alkyl chain.

**5.** Compounds according to Claim 1, where the radical X is a group $NR^{10}R^{11}$ in which $R^{10}$ and $R^{11}$ are identical or different and are in each case a hydrogen atom, a peptide residue, protein residue, a modified or unmodified DNA or RNA oligonucleotide residue, a modified or unmodified aptamer residue or a PNA residue.

**6.** Compounds according to Claim 1, where the radical X is a group $NR^{10}R^{11}$ in which $R^{10}$ and $R^{11}$ are identical or different and are in each case a hydrogen atom, an unbranched or branched $C_1$-$C_{60}$-alkyl radical.

**7.** Process for preparing compounds of the general formula (I) according to Claim 1, **characterized in that** compounds of the general formula (II) are reacted with compounds of the general formula (III) as shown in the following reaction scheme

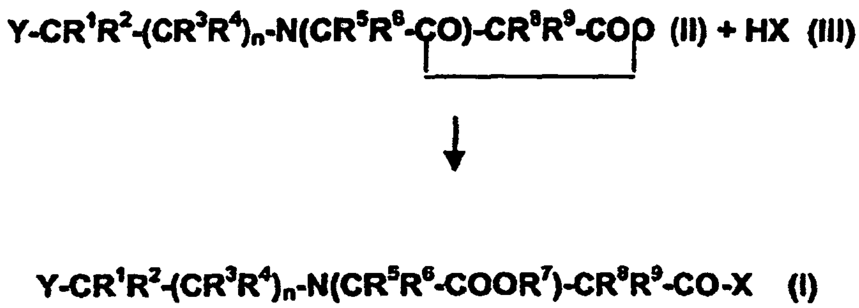
(II) + HX (III)

(I)

in which
n is the number 0, 1, or 2;
$R^1$, $R^2$, $R^3$, $R^4$, $R^5$, $R^6$, $R^7$, $R^8$ and $R^9$ have the meaning indicated above, and H in HX is a proton;
X is a radical $NR^{10}R^{11}$ in which $R^{10}$ and $R^{11}$ are identical or different and are in each case a hydrogen atom or an unbranched, branched, cyclic or polycyclic $C_1$-$C_{60}$-alkyl radical, alkenyl radical having up to 60 C atoms, polyalkenyl radical having up to 60 C atoms, alkynyl radical having up to 60 C atoms, $C_5$-$C_{60}$-polyalkynyl radical, $C_5$-$C_{60}$-aryl radical, alkylaryl radical having up to 60 C atoms or arylalkyl radical having up to 60 C atoms, which is optionally substituted by hydroxy, oxo, carboxy, aminocarbonyl, alkoxycarbonyl, amino, aldehyde or alkoxy groups having up to 20 carbon atoms, and/or is optionally interrupted and/or substituted by one or more hetero atoms from the serie O, N, S, P, As, Se; or $R^{10}$ and/or $R^{11}$ are identical or different and are in each case a hydrogen atom, a peptide residue, protein residue, a modified or unmodified DNA or RNA oligonucleotide residue, a modified or unmodified aptamer residue or a PNA residue;
Y is an $R^{12}$-S radical in which $R^{12}$ is a hydrogen atom or an unbranched, branched, cyclic or polycyclic $C_1$-$C_{60}$-alkyl radical, alkenyl radical having up to 60 C atoms, polyalkenyl radical having up to 60 C atoms, alkynyl radical having up to 60 C atoms, $C_5$-$C_{60}$-polyalkynyl radical, $C_5$-$C_{60}$-aryl radical, alkylaryl radical having up to 60 C atoms or arylalkyl radical having up to 60 C atoms, which is optionally substituted by hydroxy, oxo, carboxy, aminocarbonyl, alkoxycarbonyl, amino, aldehyde or alkoxy groups having up to 20 carbon atoms, and/or is optionally interrupted and/or substituted by one or more hetero atoms from the series O, N, S, P, As, Se; or $R^{12}$ is a peptide residue,

protein residue, a modified or unmodified DNA or RNA oligonucleotide residue, a modified or unmodified aptamer residue or a PNA residue.

**8.** Use of compounds of the general formula (I) according to Claim 1 for producing radiopharmaceutical compositions for non-invasive in vivo demonstration of receptors and receptor-containing tissue.

**9.** Pharmaceutical composition **characterized in that** it comprises a compound of the general formula (I) according to Claim 1 in solution or in lyophilized form.

**Revendications**

**1.** Composés de formule générale (I)

$$Y\text{-}CR^1R^2\text{-}(CR^3R^4)_n\text{-}N(CR^5R^6\text{-}COOR^7)\text{-}CR^8R^9\text{-}CO\text{-}X \quad (I)$$

dans laquelle
n vaut les nombres 0,1 ou 2 ;
$R^1$, $R^2$, $R^3$, $R^4$, $R^5$, $R^6$, $R^8$ et $R^9$ sont identiques et représentent hydrogène ;
$R^7$ représente un atome d'hydrogène ou un cation de métal alcalin ou alcalino-terreux ou un ion d'ammonium primaire, secondaire ou tertiaire ;
X signifie un radical $NR^{10}R^{11}$, dans lequel $R^{10}$ et $R^{11}$ sont identiques ou différents et représentent à chaque fois un atome d'hydrogène ou un radical non ramifié ou ramifié, cyclique ou polycyclique alkyle en $C_1$ à $C_{60}$, alcényle comprenant jusqu'à 60 atomes de carbone, polyalcényle comprenant jusqu'à 60 atomes de carbone, alcynyle comprenant jusqu'à 60 atomes de carbone, polyalcynyle en $C_5$ à $C_{60}$, aryle en $C_5$ à $C_{60}$, alkylaryle comprenant jusqu'à 60 atomes de carbone ou arylalkyle comprenant jusqu'à 60 atomes de carbone, qui est le cas échéant substitué par des groupes hydroxy, oxo, carboxy, aminocarbonyle, alcoxycarbonyle, amino, aldéhyde ou alcoxy comprenant jusqu'à 20 atomes de carbone et/ou le cas échéant interrompu et/ou substitué par un ou plusieurs hétéroatomes de la série O, N, S, P, As, Se ; ou $R^{10}$ et/ou $R^{11}$ sont identiques ou différents et représentent à chaque fois un atome d'hydrogène, un reste peptidique, un reste protéinique, un reste d'oligonucléotide d'ADN ou d'ARN modifié ou non modifié, un reste d'aptamère modifié ou non modifié ou un reste de PNA;
Y représente un radical $R^{12}$-S dans lequel $R^{12}$ représente un atome d'hydrogène ou un radical non ramifié, ramifié, cyclique ou polycyclique alkyle en $C_1$ à $C_{60}$, alcényle comprenant jusqu'à 60 atomes de carbone, polyalcényle comprenant jusqu'à 60 atomes de carbone, alcynyle comprenant jusqu'à 60 atomes de carbone, polyalcynyle comprenant jusqu'à 60 atomes de carbone, alkylaryle comprenant jusqu'à 60 atomes de carbone, arylalkyle en $C_5$ à $C_{60}$, qui est le cas échéant substitué par des groupes hydroxy, oxo, carboxy, aminocarbonyle, alcoxycarbonyle, amino, aldéhyde ou alcoxy comprenant jusqu'à 20 atomes de carbone et/ou le cas échéant interrompu ou substitué par un ou plusieurs hétéroatomes de la série O, N, S, P, As, Se ; ou $R^{12}$ représente un reste peptidique, un reste protéinique, un reste d'oligonucléotide d'ADN ou d'ARN modifié ou non modifié, un reste d'aptamère modifié ou non modifié ou un reste de PNA;
ainsi que leurs complexes avec des radicaux tricarbonyl-technétium-I ou tricarbonyl-rhénium-I des radio-isotopes correspondants.

**2.** Composés selon la revendication 1, dans lesquels $R^7$ représente un atome d'hydrogène ou un cation alcalin ou alcalino-terreux.

**3.** Composés selon la revendication 1, dans lesquels le radical Y représente un radical $R^{12}$-S, dans lequel $R^{12}$ représente un radical alkyle en $C_1$ à $C_{60}$ non ramifié ou ramifié.

**4.** Composés selon la revendication 3, dans lesquels le radical $R^{12}$ représente une chaîne alkyle en $C_1$ à $C_{10}$.

**5.** Composés selon la revendication 1, dans lesquels le radical X représente un groupe $NR^{10}R^{11}$, dans lequel $R^{10}$ et $R^{11}$ sont identiques ou différents et représentent à chaque fois un atome d'hydrogène, un reste peptidique, un reste protéinique, un reste d'oligonucléotide d'ADN ou d'ARN modifié ou non modifié, un reste d'aptamère modifié ou non modifié ou un reste de PNA.

**6.** Composés selon la revendication 1, dans lesquels le radical X représente un groupe $NR^{10}R^{11}$, dans lequel $R^{10}$ et $R^{11}$ sont identiques ou différents et représentent à chaque fois un atome d'hydrogène, un radical alkyle en $C_1$ à $C_{60}$ non ramifié ou ramifié.

**7.** Procédé pour la préparation de composés de formule générale (I) selon la revendication 1, **caractérisé en ce qu'**on transforme des composés de formule générale (II) avec des composés de formule générale (III) selon le schéma de réaction suivant

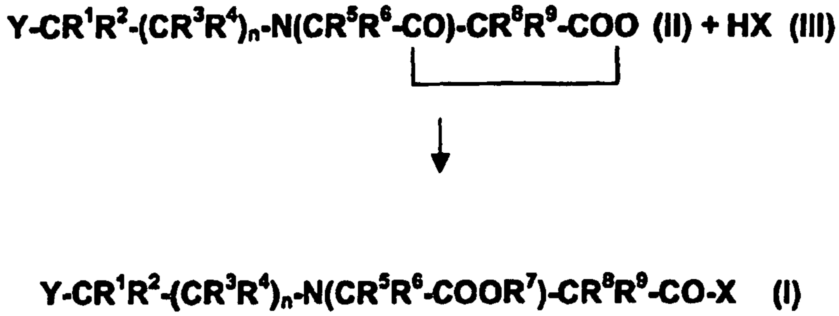

(II) + HX (III)

(I)

dans lequel
n représente les nombres 0, 1 ou 2 ;
$R^1$, $R^2$, $R^3$, $R^4$, $R^5$, $R^6$, $R^8$ et $R^9$ sont identiques ou différents et représentent hydrogène ;
$R^7$ représente un atome d'hydrogène ou un cation alcalin ou alcalino-terreux ou un ion d'ammonium primaire, secondaire ou tertiaire ;
et H dans HX représente un proton ;
X signifie un radical $NR^{10}R^{11}$ dans lequel $R^{10}$ et $R^{11}$ sont identiques ou différents et représentent à chaque fois un atome d'hydrogène ou un radical non ramifié, ramifié, cyclique ou polycyclique alkyle en $C_1$ à $C_{60}$, alcényle comprenant jusqu'à 60 atomes de carbone, polyalcényle comprenant jusqu'à 60 atomes de carbone, alcynyle comprenant jusqu'à 60 atomes de carbone, polyalcynyle en $C_5$ à $C_{60}$, aryle en $C_5$ à $C_{60}$, alkylaryle comprenant jusqu'à 60 atomes de carbone ou arylalkyle comprenant jusqu'à 60 atomes de carbone, qui est le cas échéant substitué par des groupes hydroxy, oxo, carboxy, aminocarbonyle, alcoxycarbonyle, amino, aldéhyde ou alcoxy comprenant jusqu'à 20 atomes de carbone et/ou le cas échéant interrompu et/ou substitué par un ou plusieurs hétéroatomes de la série O, N, S, P, As, Se ; ou $R^{10}$ et/ou $R^{11}$ sont identiques ou différents et représentent à chaque fois un atome d'hydrogène, un reste peptidique, un reste protéinique, un reste d'oligonucléotide d'ADN ou d'ARN modifié ou non modifié, un reste d'aptamère modifié ou non modifié ou un reste de PNA ;
Y représente un radical $R^{12}$-S, dans lequel $R^{12}$ représente un atome d'hydrogène ou un radical non ramifié, ramifié, cyclique ou polycyclique alkyle en $C_1$ à $C_{60}$, alcényle comprenant jusqu'à 60 atomes de carbone, polyalcényle comprenant jusqu'à 60 atomes de carbone, alcynyle comprenant jusqu'à 60 atomes de carbone, polyalcynyle en $C_5$ à $C_{60}$, aryle en $C_5$ à $C_{60}$, alkylaryle comprenant jusqu'à 60 atomes de carbone ou arylalkyle comprenant jusqu'à 60 atomes de carbone, qui est le cas échéant substitué par des groupes hydroxy, oxo, carboxy, aminocarbonyle, alcoxycarbonyle, amino, aldéhyde ou alcoxy comprenant jusqu'à 20 atomes de carbone et/ou le cas échéant interrompu et/ou substitué par un ou plusieurs hétéroatomes de la série O, N, S, P, As, Se; ou $R^{12}$ représente un reste peptidique, un reste protéinique, un reste d'oligonucléotide d'ADN ou d'ARN modifié ou non modifié, un reste d'aptamère modifié ou non modifié ou un reste de PNA.

**8.** Utilisation de composés de formule générale (I) selon la revendication 1 pour la préparation de compositions radiopharmaceutiques pour la représentation in vivo, non invasive de récepteurs et de tissus contenant des récepteurs.

**9.** Agent pharmaceutique, **caractérisé en ce qu'**il contient un composé de formule générale (I) selon la revendication 1 en solution ou sous forme lyophilisée.